# EUROPEAN PATENT SPECIFICATION

(11) **EP 2 389 140 B1**
(45) Date of publication and mention of the grant of the patent: **18.09.2019**
(21) Application number: 09775523.5
(22) Date of filing: 22.12.2009
(51) Int. Cl.: A61F 2/38, A61F 2/00, A61F 2/30

(54) **KNEE PROSTHESIS**
KNIEPROTHESE
PROTHÈSE DE GENOU

(30) Priority: 23.12.2008 US 140183 P; 15.06.2009 US 484594
(43) Date of publication of application: 30.11.2011
(73) Proprietor: Aesculap Implant Systems, LLC, Center Valley, PA 18034 (US)
(72) Inventor: MIHALKO, William, Germantown, TN 38138 (US); SALEH, Khaled, J., Springfield, IL 62711 (US); MOUSSA, Said, 52000 Chamrandes (FR); MOUILLET, Dominique, 52000 Semoutiers (FR)
(74) Representative: Hoeger, Stellrecht & Partner Patentanwälte mbB
(86) International application number: PCT/US2009/069163
(87) International publication number: WO 2010/075365

(56) References cited:
- EP-A2- 0 941 719
- EP-A2- 1 591 082
- WO-A1-2004/058108
- WO-A2-2007/119173
- GB-A- 2 253 147
- US-A- 5 549 686
- US-A- 6 013 103
- US-A1- 2007 135 925

## Description

This application claims the benefit of earlier filed U.S. Provisional Application Serial No. 61/140,183 filed December 23, 2008 and U.S. Non-provisional Application Serial No. 12/484,594 filed June 15, 2009.

### Field of Invention

The field of invention relates to artificial joints, and more particularly to a knee prosthesis in accordance with the preamble of claim 1.

### Background

As is the case with many joint prostheses or replacements, replicating natural anatomical movement through artificial mechanical devices proves challenging. This is true especially with the knee, which allows for relative complex movement and kinematics between the femoral condyles and the tibia. This relative motion is complex in that it accounts for both rolling and sliding between the contact surfaces at varying rates throughout the flexion arc. Along with such movement during knee bending is a rotational movement between the tibia and femur. As such, knee prostheses have historically tried to replicate the full range of knee movement, throughout and between full flexion and extension in all planes (coronal-varus/valgus, sagittal-flexion, transverse-rotation). True anatomical movement would allow rollback and translation of the femoral condyles on the tibia, all while also allowing rotational movement during flexion/extension.

Prior art designs have included femoral components with cams and tibial components with posts. It has been disclosed that an asymmetrical cam can be utilized to cause rotation between the two components. These designs, however, have taught architectures that require relatively high posts to support upward movement of the cam during flexion and/or rely on the anterior-posterior position of the post and cam. Other prior art designs have disclosed the use of tibial bearing surfaces to cause rotation between the components. These designs, however, have taught architectures that require tibial components that are asymmetrical in the lateral-medial cross-section.

US 2007/0135925 A1 discloses a surface guided knee replacement. An artificial knee joint is known from EP 1 591 082 A2. WO 2004/058108 A1 relates to high performance knee prostheses. A knee prosthesis with a tapered cam is disclosed in US 5,549,686. EP 0 941 741 A2 describes a four compartment knee. A medial pivot knee prosthesis is known from US 6,013,103. Further knee prostheses having the features of the preamble of claim 1 are disclosed in WO 2007/119173 A2 and GB 2 253 147 A.

### Summary of Invention

The present invention identifies and overcomes a further shortcoming of the prior art. As stated above, true anatomical movement would allow rollback and translation of the femoral condyles on the tibia, all while also allowing rotational movement during flexion/extension. It has now been identified that knee prosthesis designs, which utilize bearing surfaces to allow flexion/extension and enable rotational movement, often exhibit a forward translation of the femoral component upon the tibial component. This phenomenon, called "paradoxical roll forward", prevents the knee prosthesis from accurately replicating the anatomic movement of a natural knee.

It is an object of the present invention to overcome the disadvantages of the prior art.

This object is achieved by a knee prosthesis as defined in claim 1. Further preferred embodiments of the invention are defined in the dependent claims.

Embodiments of the present invention prevent paradoxical roll forward and enable true anatomical movement of the knee.

The present invention provides a knee prosthesis having a femoral component with two condyles with an opening disposed between the two condyles. Also included is a tibial component having bearing surfaces to support each of the femoral component condyles. The femoral component and tibial component are engageable by contact between the femoral condyles and tibial bearing surfaces. By moving the femoral and tibial components in flexion from about 0° to about 165°, the contact regions between the femoral component and the tibial component cause rotation between the tibial and femoral components.

A natural knee contains a posterior cruciate ligament which helps provide stability and strength to the knee joint. This ligament may become damaged or ruptured and, thus, no longer provide support to the knee joint. The present invention provides prostheses which employ cruciate-substituting or cruciate-retaining knee prosthesis architectures for achieving kinematic knee motion. Embodiments of the present invention contain components which function to substitute for damaged or ruptured posterior cruciate ligament. Other embodiments of the present invention work to supplement the cruciate ligament when it is healthy enough to be retained in the knee prosthesis.

A knee prosthesis not forming part of the invention as claimed includes a femoral component with two condyles with an opening disposed between the two condyles, and a cam extending between the condyles forming a posterior boundary to the opening. The knee prosthesis also includes a tibial component having bearing surfaces to support each of the femoral component condyles, and a post disposed between the bearing surfaces and extending superior from the tibial component. The femoral component and tibial component are engageable by contact between the femoral condyles and tibial bearing surfaces, and by contact between the cam and post during at least a portion of flexion between the femoral and tibial components. By moving the femoral and tibial components in flexion from about 45° to about 165°, the contact region between the cam and post moves inferiorly and medially to cause medial rotation between the tibial and femoral components.

Another knee prosthesis includes a femoral component with two condyles, with an opening disposed between the two condyles, and a tibial component having bearing surfaces to support each of the femoral component condyles. The medial and lateral femoral condyles are asymmetrical in an anterior-posterior cross-section. The lateral condyle has a similar architecture of the lateral condyle of the first knee prosthesis but the medial condyle of this knee prosthesis has at least one region of uniform radius. Similarly, the medial and tibial bearing surfaces are asymmetrical in an anterior-posterior cross-section. The femoral component and tibial component are engageable by contact between the femoral condyles and tibial bearing surfaces. However, the lateral and medial bearing surfaces remain symmetrical in the lateral-medial cross-section. During flexion of the femoral and tibial components from about 0° to about 90°, a period of flexion in which other knee designs are prone to paradoxical roll forward, an anterior lip of the medial tibial bearing surface functions to prevent roll forward of the femoral component of the present knee prosthesis. The anterior-posterior design architecture of the medial tibial bearing surface constrains the translation of the femoral component and works with the uniform radius of curvature of the medial femoral condyle to also prevent the paradoxical roll forward. The asymmetrical lateral and medial femoral condyles, and the asymmetric lateral and medial tibial bearing surfaces enable the condyles to translate posteriorly at different rates and cause rotation between the tibial and femoral components. Thus, by moving the femoral and tibial components in flexion from about 0° to about 165°, the contact regions between the femoral component and the tibial component prevent roll forward, enable roll back, and cause rotation between the tibial and femoral components.

### Brief Description of the Figures

Fig. 1 illustrates a tibial component;
Fig. 2 illustrates a femoral component;
Fig. 3 illustrates a tibial component (with a stem) and a femoral component mated together;
Fig. 4 illustrates a partial cross-sectional view of the prosthesis at about 0° flexion;
Fig. 5 illustrates a partial cross-sectional view of the prosthesis at about 45° flexion;
Fig. 6 illustrates a partial cross-sectional view of the prosthesis at about 90° flexion;
Fig. 7 illustrates a series of cross-sectional views at three planes of interaction of the cam and post of the prosthesis;
Fig. 8 illustrates a partial cross-sectional view of the prosthesis at about full flexion;
Fig. 9 illustrates that of Fig. 8 but with the addition of a patellar implant;
Fig. 10 illustrates a top down view of the prosthesis at about 45° flexion;
Fig. 11 illustrates a partial cross-sectional view from the top down of the prosthesis at about 90° flexion;
Fig. 12 illustrates a partial cross-sectional view from the top down of the prosthesis at about 145° flexion;
Fig. 13 illustrates a top down view of the prosthesis of an embodiment of the prosthesis at about 145° flexion; and
Fig. 14 illustrates a cross-sectional view at high flexion showing separation of the lateral condyle from tibial component;
Fig. 15 illustrates a tibial component in accordance with an embodiment of the present invention;
Fig. 16 illustrates a femoral component in accordance with the embodiment of the present invention;
Fig. 17 illustrates a lateral-medial cross-sectional view of a tibial component in accordance with the embodiment of the present invention;
Fig. 18a illustrates an anterior-posterior cross-sectional view of a tibial component in accordance with the embodiment of the present invention;
Fig. 18b illustrates a reverse view of the anterior-posterior cross-sectional view of Fig. 18a.;
Fig. 19a illustrates a partial cross-sectional view of a medial femoral condyle of the embodiment of the present invention at about 0° flexion;
Fig. 19b illustrates a partial cross-sectional view of a lateral femoral condyle of the embodiment of the present invention at about 0° flexion;
Fig. 20 illustrates a partial cross-sectional view of the prosthesis of the embodiment of the present invention at about 0° flexion;
Fig. 21 illustrates a partial cross-sectional view of the prosthesis of the embodiment of the present invention at about 45° flexion;
Fig. 22 illustrates a partial cross-sectional view of the prosthesis of another embodiment of the present invention at about 90° flexion;
Fig. 23 illustrates a top down view of the prosthesis of the embodiment of the present invention at about 45° flexion;
Fig. 24 illustrates a partial cross-sectional view from the top down of the prosthesis of the embodiment of the present invention at about 90° flexion;
Fig. 25 illustrates a partial cross-sectional view from the top down of the prosthesis of the embodiment of the present invention at about 145° flexion.

### Detailed Description of the Invention

The present invention provides a knee prosthesis which allows for anatomically correct knee movement. It does so by providing an upper, or femoral, component which is designed to mechanically interact with a lower, or tibial, component to achieve kinematic movement consistent with a natural knee joint. Generally, the two pieces interact by providing several different contact surfaces, not all of which are engaged between the two components of the knee throughout the range of motion.

Two such contact surfaces are the load bearing condylar surfaces between the femoral component and the tibial component. These surfaces are defined by medial and lateral condylar surfaces which are referred to as the load bearing surfaces for a given knee joint. Specifically, a medial load bearing surface is defined between the medial femoral condyle and its counterpart on the tibial component, namely a medial tibial accommodating surface. Likewise, a lateral load bearing surface is defined between the lateral femoral condyle and its counterpart on the tibial component, namely a lateral tibial accommodating surface.

In an embodiment of a knee prosthesis not forming part of the invention as claimed, a different contact surface also exists to cause rotational movement between the femoral and tibial components, during certain degrees of knee extension/flexion which will allow for a kinematic pattern that more closely resembles that of the natural knee. This contact surface is defined by interaction between a post on the tibial component (preferably polyethylene) and a cam surface on the femoral component (preferably metallic). Because the point of contact between the femoral condyles and their corresponding tibial load-receiving components changes in an anterior/posterior direction (that is to say there is front/back translation of the point of contact) during knee movement, the post and cam do not interact during all degrees of knee flexion. Instead, the post and cam only interact during those points of knee movement for which they are designed to cause a replicated natural knee kinematic envelop. This interaction occurs when the anterior/posterior movement of the femoral/tibial contact causes the post and cam to engage, or when flexion of the knee causes enough rollback of the femoral component to engage the tibial post against the cam of the femoral component.

It should be noted, however, that once flexion typically reaches about 45°, anterior/posterior translation does not stop but occurs at different rates in the medial and lateral compartments of the knee. Moreover, as the knee bends, the lateral condyle rolls back to a position of about 10-15mm posterior at about 120° flexion, but the medial condyle rolls back only about 4-5mm to a final position of about 1-3mm posterior. This difference in posterior movement in the two compartments of the knee is seen as rotation of the femoral component on the tibial component, and occurs with continued rollback of the femoral condyles. This interaction of the post and cam, as well as the movement of the femoral condyles with respect to the tibial bearing surfaces will be addressed below.

The movement described is achieved through the architecture of the both the femoral component, the tibial component, and in particular the cam and post dimensions. All of these aspects are integrated into a system which provides for sophisticated, anatomical movement within the prosthetic knee.

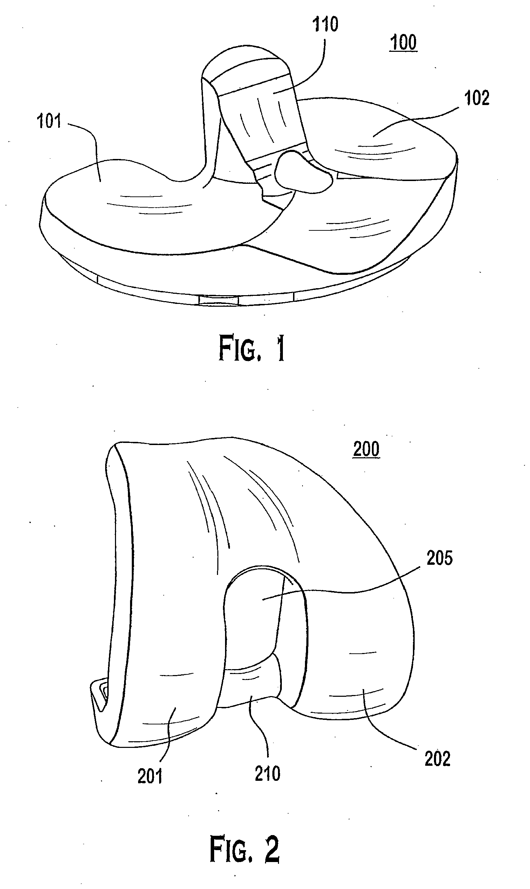

Fig. 1 shows a tibial component 100 not forming part of the invention as claimed. This tibial component 100 has two load bearing surfaces, shown as load bearing surface 101 and load bearing surface 102. For a right knee joint, load bearing surface 101 would be the lateral condyle load bearing surface, and load bearing surface 102 would be the medial condyle load bearing surface. Post 110 is shown extending upward, or in a superior direction, from the lateral plane generally defining the tibial insert. Post 110 will be described in more detail below.

Fig. 2 illustrates a femoral component 200 not forming part of the invention as claimed. Cam 210 is shown bridging a gap between the femoral condyles 201 and 202. Opening 205 is defined by the condyles 201 and 202 which extend anteriorly around the side of the opening opposite cam 210. Cam 210 is generally disposed in a posterior portion of the opening in the femoral component. Cam 210 and its dimensions will be defined in more detail below.

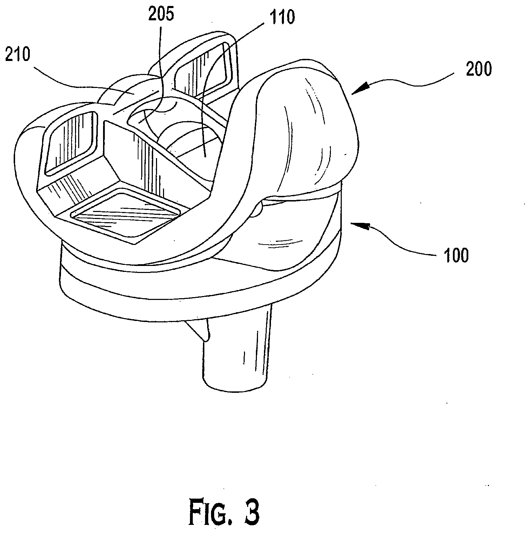

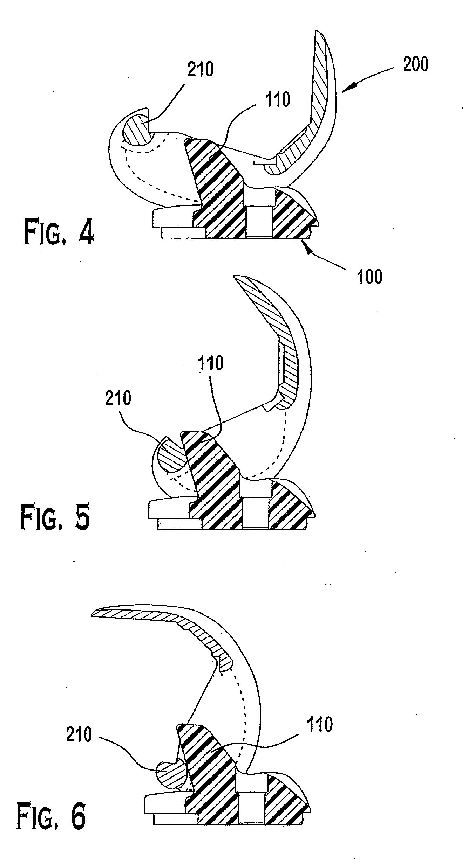

Fig. 3 shows femoral component 200 disposed atop tibial component 100. Post 110 is shown extending through opening 205. Fig. 3 shows the components in a position of 0° flexion. As can be seen from Fig. 3, cam 210 is not in contact with post 110 at this point. It is also noteworthy that in this position, there is no contact between the anterior surface of post 110 and the anterior boundary of opening 205. This aspect can be seen perhaps more clearly in Fig. 4, which shows a partial cross-sectional view of that shown in Fig. 3. This aspect is important because it reduces wear on the tibial post 110.

For an example of an implant having both anterior and posterior cams, see U.S. Patent No. 6,325,828, which illustrates a femoral component having a blind hole or slot/recess (as opposed to an opening) bordered by cams on both sides (anterior and posterior). As such, and as explicitly disclosed, the anterior cam engages the post at full extension (or 0° flexion).

As the knee bends toward a flexion of about 45°, cam 210 moves toward post 110 as anterior translation occurs between the contact region of the femoral condyles and their respective load bearing surfaces on tibial component 100. The orientation of the two components, and in particular the cam and post, at 45° flexion, is illustrated in Fig. 5, which shows a partial cross-sectional view of the components at about 45° flexion. At this point in the knee movement, the cam 210 has contacted post 110 and as further flexion occurs, the rotational movement caused by the interaction of the post and cam causes slight medial rotation of the femoral component with respect to the tibial component.

Fig. 6 shows the partial cross-section of the two components at about 90° flexion. Note that the contact point between the cam and post moves downward along the post, or inferiorly, as flexion increases. This is due to the architecture of the cam and post and is designed as a part of the knee movement based on the anatomical requirements of the natural knee joint.

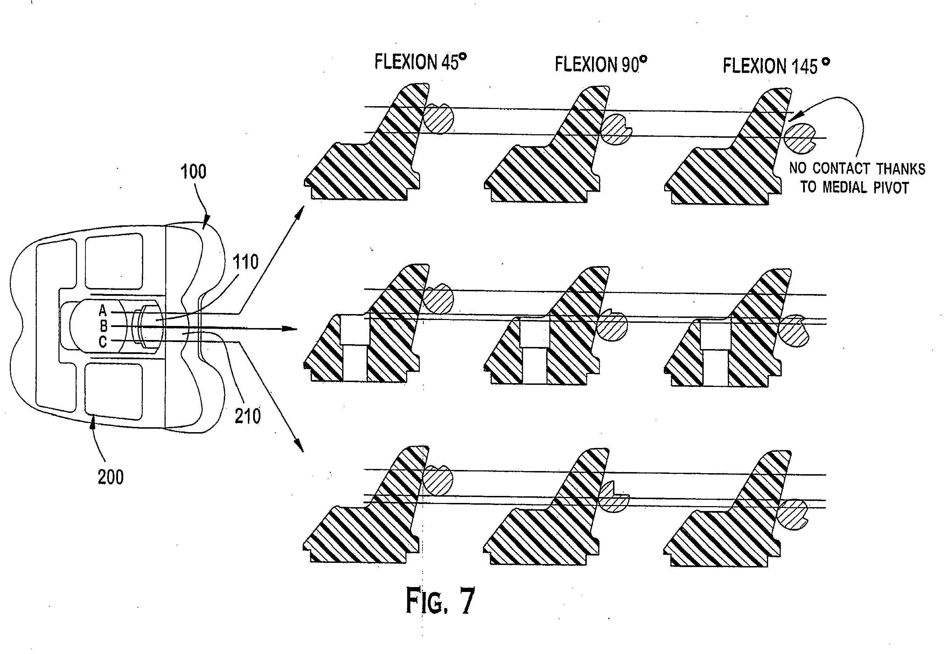

Further defining this aspect of the invention is Fig. 7. Fig. 7 shows the cross-sections of three different planes at three different angles of flexion. Planes A, B, and C are shown and illustrate the asymmetry of the cam 210 and the effect of that asymmetry on the rotation and inferior movement of the cam down the post as flexion increases. At 45° flexion, plane A indicates contact of the cam and post at a point relatively high on the post. As flexion increases to 90°, the cam is working its way down the post as the femoral component rotates medially with respect to the tibial component. At 145° flexion, not only has the cam moved further downward along the posterior side of the post (at planes B and C), but in fact, at plane A, or the lateral side of the cam, the cam has disengaged the post altogether as medial rotation has separated the cam from the post at this point. Thus, there is seen a medial rotation consistent with natural knee movement while the cam has actually moved down along the post. Stability is one advantage of the implant designed this way in accordance with the invention.

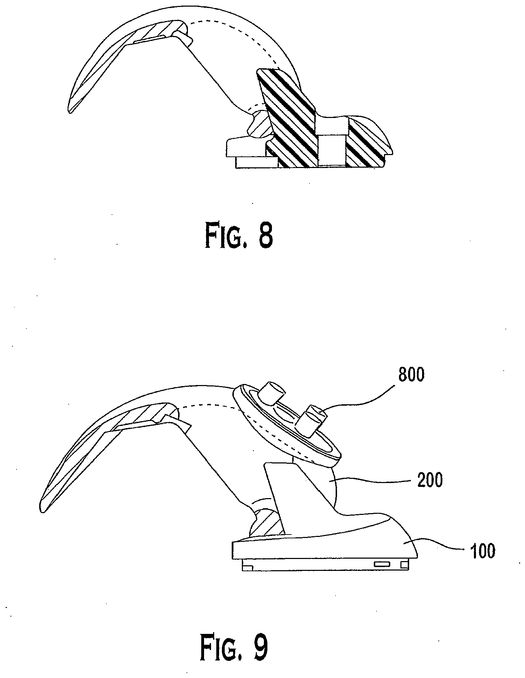

This later point is important to achieve natural knee movement with respect to a patellar implant. Fig. 8 shows knee prosthesis of the present invention at about 145° flexion. At this point, and as noted above, the cam has moved downward along the post. The post therefore only needs to be as high as is necessary to engage the cam at the first point of contact, namely at about 45° flexion (because after that the cam moves downward).

The relative shortness of the post is important because it allows for clearance of the patellar implant as shown in Fig. 9. There, patellar implant 800 is shown disposed on femoral component 200. Unlike prior art designs that have upward cam movement during flexion, and therefore require higher posts to extend upward from the initial point of contact, the present invention is configured to provide downward cam movement and therefore relatively shorter posts are necessary. This allows for patellar clearance during knee rotation as shown in Fig. 9.

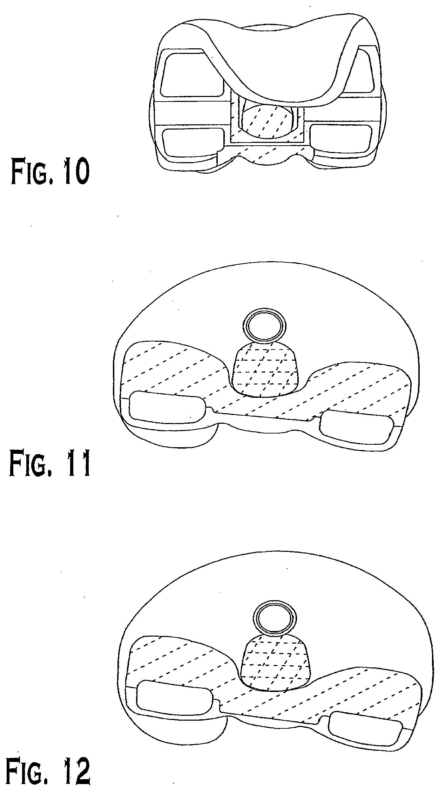

By way of further illustration, Figs. 10-12 show a top-down partial cross-sectional view of the prosthesis during flexion of 45°, 90°, and 145°, respectively. As can be seen from these views, the cam has a shape and size quite different on the lateral side than on its medial side. This cam and its particular shape and orientation provides an angled surface which acts with the post to drive a very precise medial pivot and femoral rotation in the transverse plane.

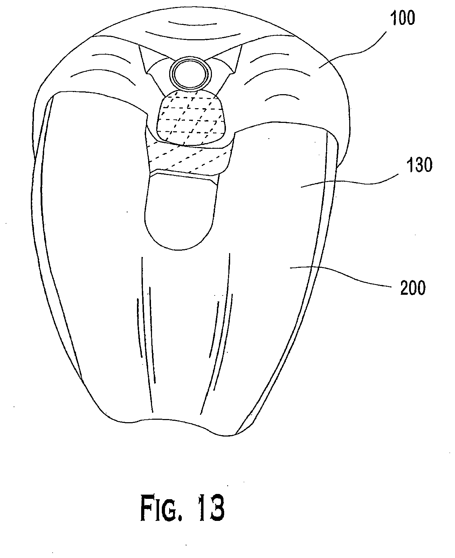

Fig. 13 illustrates a top down view of the cam and post interaction and also shows the medial rotation of the femoral component with respect to its tibial component. Note that even at this relatively high flexion, the cam is disposed somewhat under the post and enlarges in cross-sectional area toward the lateral end of the cam where it abuts the lateral condyle 130.

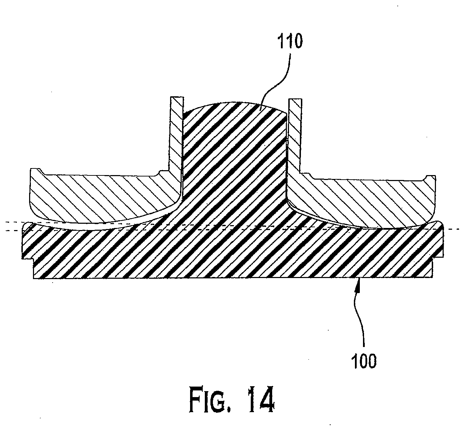

It is also noteworthy that the post may be sized to permit or restrict separation of the lateral condyle from the tibial component, which may occur due to varus-valgus moments and may be necessary to replicate a natural knee movement. See, for example, Fig. 14, which shows separation of the lateral condyle 140 from tibial component 100. This separation is due, in part, to the architecture of the cam and the post to which it engages during flexion. Fig. 14 shows an optional increased post width design in the medial direction, which can be used to support varus-valgus moments. The post width can be sized to resist such varus-valgus motion between the femoral and tibial components, such as in the case of diminished soft tissue strength in the knee joint. In such cases, the post can be sized to provide additional strength and support to the knee joint. Conversely, the post width can be sized to allow some degree of varus-valgus motion to replicate anatomically correct movement.

One advantage to the prosthesis is that it allows for less soft tissue strain by allowing for more anatomical movement instead of equal rollback in both compartments of the tibial insert. This design gives three advantages over previous designs: 1) less soft tissue strain due to more anatomical movement, 2) better natural motion replication in the medial compartment without increasing constraint, and 3) decreased tibial strain with no edge loading in the medial compartment. Although the above illustrations show knee flexion at 0°, 90°, and 145°, the range of motion allowed for in the design would be at least -10° (hyperextension) to about 165° (high flexion) with supported articulation in the medial and lateral compartments of the knee.

Moreover, as flexion continues beyond 45°, anterior/posterior translation continues to occur, but is guided by the post/asymmetric-cam interaction. Because of the relative dimensions of the post, and in particular the type of asymmetrical cam on the femoral component, proper rotational movement between the femoral component and tibial component is achieved.

Consistent with that described above, the interaction between the tibial component post and the femoral component tapered asymmetric cam, is designed to preferably begin at 45° flexion. It should be noted that the interaction can be controlled through manipulation of the dimensions of the post and cam. This is accomplished through varying the cross-sectional dimensions of the cam from a medial to lateral direction, with the lateral portion of the cam being generally larger than the medial portion. More specifically, the largest cross-sectional area of the cam occurs where the cam meets the lateral condyle. Moving in a medial direction, the cam tapers in a manner consistent with that which causes kinematic rotation as the knee bends past 45° flexion.

It is also noteworthy that there is no interaction between the post and cam at full extension. This prevents unnecessary wear on the tibial post which would otherwise weaken it over time and could even result in failure (i.e, it could shear off).

It has been identified that existing knee prosthesis designs, which utilize bearing surfaces to allow flexion/extension and enable rotational movement, exhibit a paradoxical roll forward of the femoral component upon the tibial component. This forward translation is unlike the anatomic movement of a natural knee. Embodiments of the present invention prevent such paradoxical roll forward and enable true anatomical movement of the knee.

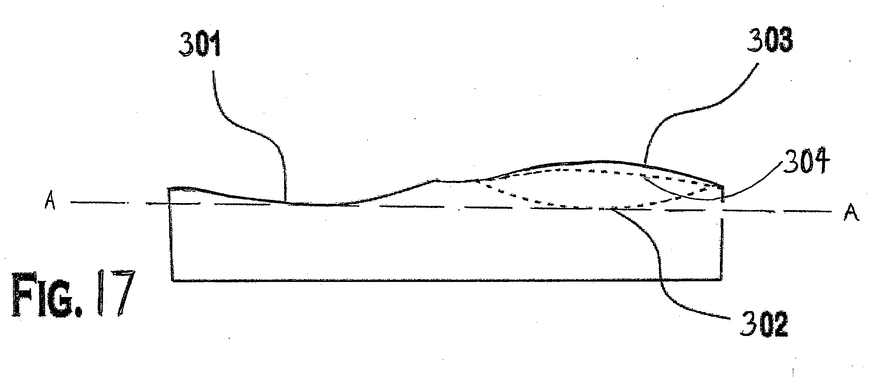

In one such embodiment of the present invention, rotational movement between the femoral and tibial components is enabled by contact surface interaction between asymmetrical medial and lateral femoral condyles and their respective asymmetrical medial and lateral tibial bearing surfaces. In this embodiment, the lateral and medial condyles of the femoral component are asymmetrical such that the medial femoral condyle has at least one region of uniform radius and the lateral femoral condyle does not. The anterior and posterior lips of the medial tibial bearing surface project higher from the tibial component than the anterior and posterior lips of the lateral tibial bearing surface, creating asymmetry of the tibial component. However, the lateral and medial bearing surfaces remain symmetrical in the lateral-medial cross-section. This can be seen, for example, in Fig. 17.

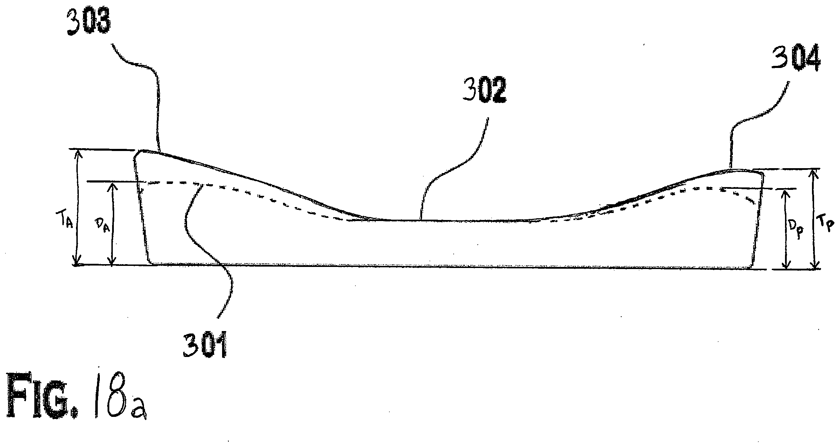

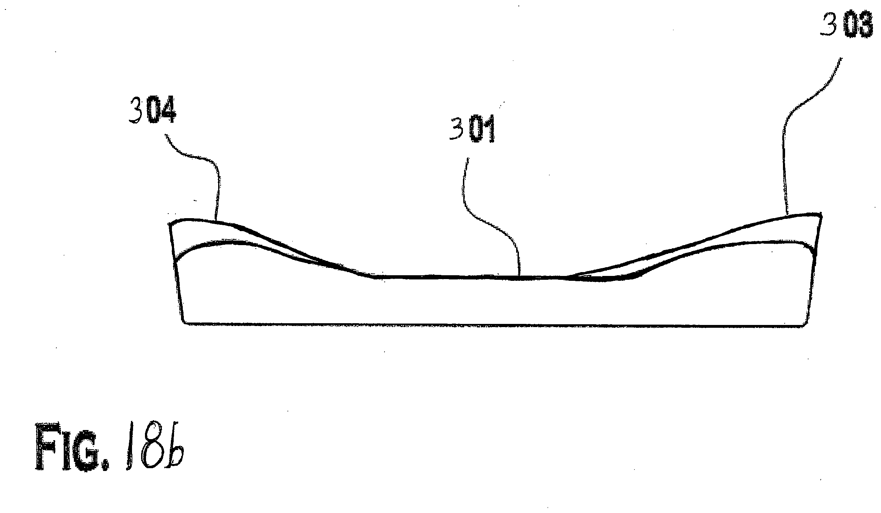

During flexion of the femoral and tibial components from about 0° to about 90°, the anterior lip of the medial tibial bearing surface and a first region of uniform radius of curvature of the medial femoral condyle function to retain the medial femoral condyle at an effectively constant contact point upon the medial tibial bearing surface. This architecture further prevents roll forward of the medial femoral condyle on the medial tibial bearing surface. The lateral tibial bearing surface is less constrained in the anterior-posterior architecture to allow roll back to occur at an earlier degree of flexion than upon the medial tibial bearing surface. While the higher lips of the medial tibial bearing surface cause it to be asymmetrical with the lateral tibial bearing surface, the tibial component remains symmetrical in its minimum thickness along a medial-lateral cross-section. This minimum thickness symmetry of the tibial component allows the contact points of the medial and lateral condyles with their respective bearing surfaces, at 0° flexion, to be at equal elevations from the bottom of the tibial component. This is detailed further, for example, in Figures 17, 18a, and 18b. The medial lips retain the medial condyle within this area of the tibial bearing surface having symmetrical thickness during flexion from about 0° to about 90°. The point of contact between the femoral condyles and their corresponding tibial load-receiving components changes in an anterior/posterior direction (that is to say there is front/back translation of the point of contact) during knee movement. However, the asymmetrical structures of the lateral femoral condyle and lateral tibial bearing surface (vis-à-vis their respective medial counterparts) allow the lateral condyle to posteriorly translate upon the lateral bearing surface further. This disparity in posterior translation of the lateral and medial condyles is seen as rotation of the femoral component on the tibial component. Rotational movement, posterior translation, and other kinematic motion of this embodiment is therefore achieved without a femoral cam, a tibial post, or a post/cam contact surface. This novel functionality of this embodiment of the present invention is useful in knee replacement procedures, especially in cruciate-retaining procedures where such functionality has not been possible prior to the present design.

Once flexion reaches about 90°, anterior/posterior translation of the condyles does not stop but occurs at a different rate in the medial and lateral compartments of the knee. During deep flexion from about 90° to about 120°, a second region of uniform radius of the medial femoral condyle may be utilized to engage the anterior lip of the medial tibial bearing surface. Similarly, a third region of uniform radius of the medial femoral condyle can be used to engage the tibial component from about 120° to about 165° of flexion. These regions of uniform radius further enable rollback of the femoral condyles and rotation of the femoral component on the tibial component. Thus, by moving the femoral and tibial components in flexion from about 0° to about 165°, the contact regions between the femoral component and the tibial component prevent roll forward as well as cause rotation between the tibial and femoral components.

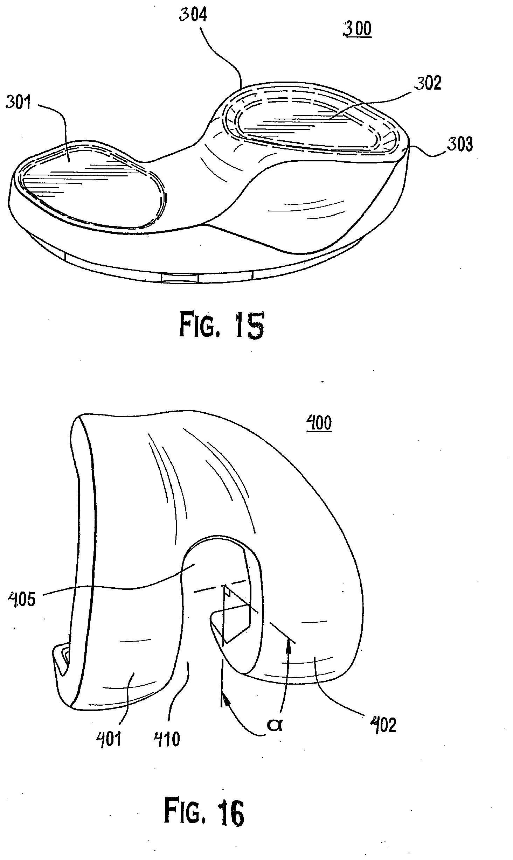

Fig. 15 shows a tibial component 300 in accordance with an embodiment of the present invention. This tibial component 300 has two load bearing surfaces, shown as load bearing surface 301 and load bearing surface 302. For a right knee joint, load bearing surface 301 would be the lateral condyle load bearing surface, and load bearing surface 302 would be the medial condyle load bearing surface. Anterior lip 303 and posterior lip 304 project from medial condyle bearing surface 302, Anterior lip 303 and posterior lip 304 are defined in more detail below.

Fig. 16 illustrates a femoral component 400 in accordance with an embodiment of the present invention. Opening 410 is defined by the condyles 401 and 402 which extend anteriorly around the side of the opening 410. In this illustration, condyle 401 is the lateral femoral condyle and condyle 402 is the medial femoral condyle. Condyles 401 and 402 are defined in more detail below.

Fig. 17 illustrates a lateral-medial cross-sectional view of a tibial component in accordance with this embodiment of the present invention. Line "A" of Fig. 17 shows that the lateral and medial bearing surfaces 301 and 302, respectively, are symmetrical and of equivalent thickness (or, elevation) from the bottom of the tibial component. Anterior lip 303 and posterior lip 304 of the medial bearing surface 302 show the asymmetrical architecture of the tibial component, vis-à-vis the lateral side of the tibial component. This can also be seen in Fig. 15. In this embodiment of the present invention, at about 0°, the medial and lateral femoral condyles would engage the tibial component at bearing surfaces 301 and 302, at contact points along line "A" (shown in Fig. 17). As the knee bends in flexion from about 0° to about 90°, an anterior lip 303 of the medial tibial bearing surface 302 functions to retain the medial femoral condyle at an effectively constant contact point upon the tibial bearing surface and interacts with a region of the medial femoral condyle having a uniform radius of curvature to prevent roll forward of the femoral component. The architecture of the lateral tibial bearing surface does not contain this constraint and engages the non-uniform radius of the lateral femoral condyle to enable roll back.

Fig. 18a illustrates an anterior-posterior cross-sectional view of a tibial component in accordance with this embodiment of the present invention, while Fig. 18b illustrates a reverse view of this same cross-section. The medial tibial bearing surface 302 has projections extending superiorly from the tibial component which comprises an anterior lip and a posterior lip. Anterior lip 303 has a thickness T_{A} and posterior lip 304 has a thickness T_{P}. In one embodiment of the present invention, thicknesses T_{A} and T_{P} have a proportional ratio of about 7 : 3. The lateral tibial bearing surface 301 has an anterior lip dimension D_{A}, and a posterior lip dimension D_{P}. In one embodiment of the present invention, dimensions D_{A} and D_{P} have a proportional ratio of about 4 : 2. As detailed, the medial bearing surface 302 includes higher lip projections than the lateral bearing surface 301. In this example, the anterior lips of the medial and lateral bearing surfaces have about a 7 : 4 ratio while the posterior lips have about a 3 : 2 ratio.

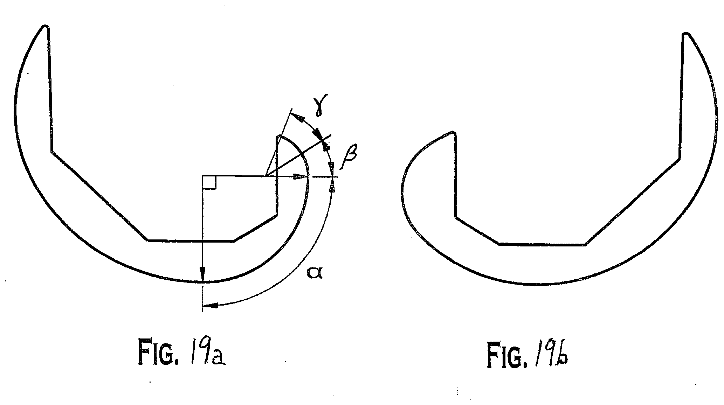

Fig. 19a and Fig. 19b illustrate partial cross-sectional views of the medial femoral condyle and the lateral femoral condyle, respectively, of another embodiment of the present invention at about 0° flexion. The architecture of the lateral femoral condyle has a changing radius at its distal end, while the medial femoral condyle has at least one region of uniform radius at its distal end. In the embodiment shown in Fig. 19a, the medial condyle has a first region of uniform radius α, a second region of uniform radius β, and a third region of uniform radius γ. The lateral condyle is asymmetrical from the medial condyle in that it does not possess some or all of these regions of uniform radius, as is shown in Fig. 19b.

During flexion of the femoral and tibial components from about 0° to about 90°, the contact point beween the femoral and tibial components is in this first region of uniform radius α. This uniform radius design of the medial condyle combines with the anterior lip architecture of the medial bearing surface to prevent roll forward of the femoral component of the present knee prosthesis. Additionally, the asymmetrical lateral and medial femoral condyles, and the asymmetric lateral and medial tibial bearing surfaces enable the condyles to translate posteriorly at different rates and cause rotation between the tibial and femoral components. Optionally, the medial condyle may include a second region of uniform radius β to engage the medial bearing surface in flexion from about 90° to about 120°, and a third region of uniform radius γ to engage the medial bearing surface in flexion from about 120° to about 165°. The second region of uniform radius β and third region of uniform radius γ may assist further in the purposes of this invention. Thus, by moving the femoral and tibial components in flexion from about 0° to about 165°, the contact regions between the femoral component and the tibial component prevent roll forward, enable roll back, and cause rotation between the tibial and femoral components.

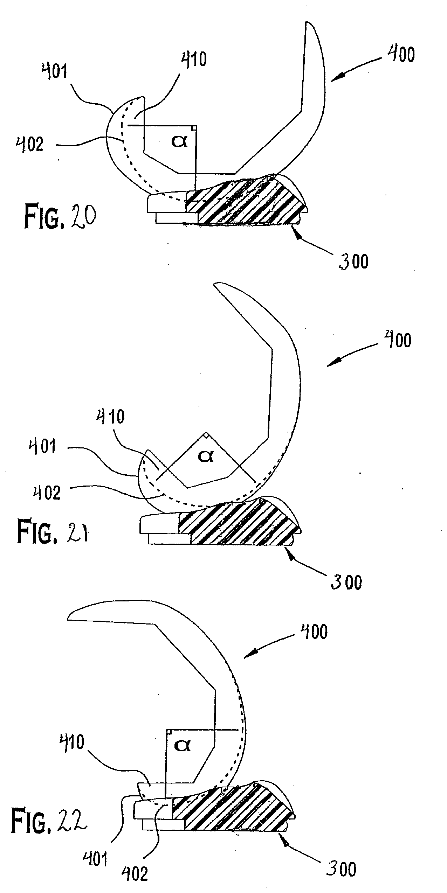

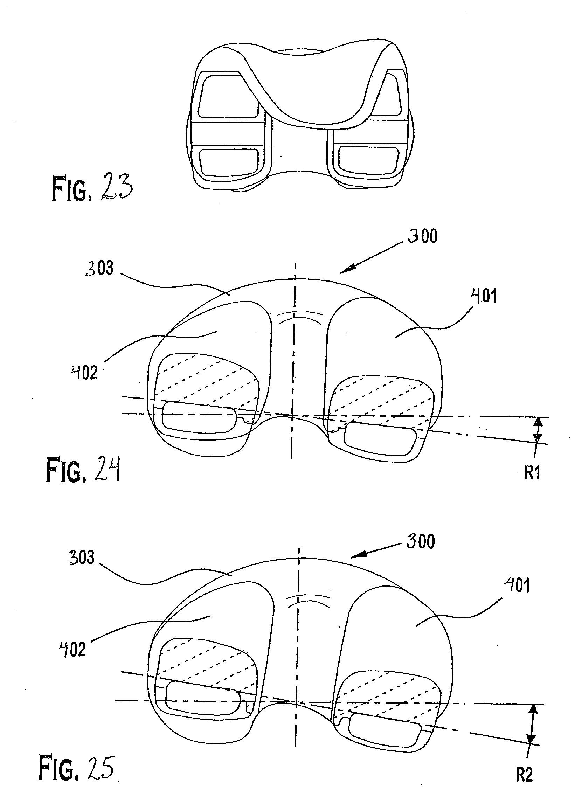

The interaction of the femoral condyles with the tibial bearing surfaces, at different amounts of flexion, is illustrated in Fig. 20, Fig. 21, and Fig. 22. As the knee bends toward a flexion of about 90°, the contact regions between the femoral condyles of femoral component 400 and their respective load bearing surfaces on tibial component 300 change. The orientation of the two components is illustrated in Fig. 20, which shows a partial cross-sectional view of the components at about 0° flexion. At this point in the knee movement, the medial femoral condyle 402 is retained at an effectively constant contact point upon its respective bearing surface of medial component 300. As further flexion occurs, illustrated in Fig. 21, medial femoral condyle 402 and lateral femoral condyle 401 translate posteriorly at different rates, causing rotational movement of the femoral component 400 upon the tibial component 300. Fig. 22 shows the partial cross-section of the two components after further knee flexion. Note that the contact points between femoral condyles 402 and 401 and their respective tibial bearing surfaces continue to translate posteriorly as flexion increases. This is due to the architecture of this embodiment, which is designed as a part of the knee movement based on the anatomical requirements of the natural knee joint.By way of further illustration, Figs. 23-25 show a top-down partial cross-sectional view of the prosthesis during flexion of 45°, 90°, and 145°, respectively. Figs. 24 and 25 illustrate this rotation during knee flexion from about 90° to about 145°. Tibial component 300 includes a higher projection on the medial anterior bearing surface, creating a medial anterior lip 303. Fig. 24 illustrates the knee prosthesis at about 90° flexion, where the femoral component has rotation R1 upon the tibial component. Fig. 25 illustrates the knee prosthesis at about 145° flexion, where the femoral component has rotation R2 upon the tibial component. Medial condyle 402 and lateral condyle 401 interact tibial component 300 at contact points. Although these further illustrations show knee flexion at 0°, 90°, and 145°, the range of motion allowed for in the embodiments of the present invention would be at least -10° (hyperextension) to about 165° (high flexion), with supported articulation in the medial and lateral compartments of the knee.

As can be seen from these views, this embodiment of the invention causes rotational movement, posterior translation, and other kinematic motion without a femoral cam, a tibial post, or a post/cam contact surface. The architecture of the medial and lateral femoral condyles, as well as the medial and lateral tibial bearing surfaces, drive a very precise medial pivot and femoral rotation in the transverse plane. This novel functionality of this embodiment of the present invention is useful in knee replacement procedures, especially in cruciate-retaining procedures where such functionality has not been possible prior to the present design.

## Claims

1. A knee prosthesis comprising a femoral component (400) having a medial condyle (402) and a lateral condyle (401) with an opening (410) disposed between the two condyles (402, 401), the medial condyle (402) having at least one region of uniform radius (α, β, γ) where it engages a medial bearing surface (302) during flexion from about 0° to about 165°, and the lateral condyle (401) having a region of non-uniform radius where it engages a lateral bearing surface (301) during flexion from about 0° to about 165°, a tibial component (300) having the medial bearing surface (302) and the lateral bearing surface (301) to support each of the femoral component condyles (402, 401), the medial bearing surface (302) having an anterior projection comprising a medial anterior lip (303), and the femoral component (400) and tibial component (300) engageable at contact regions between the femoral condyles (402, 401) and tibial bearing surfaces (302, 301) such that upon moving the femoral and tibial components (400, 300) in flexion from about 0° to about 90°, the medial anterior lip (303) of the medial bearing surface (302) causes the medial condyle (402) to be retained at a constant contact point upon the medial bearing surface and prevents roll forward of the femoral component (400) while the lateral bearing surface (301) engages the non-uniform radius of the lateral condyle (401) to enable rollback of the lateral condyle on the lateral bearing surface, and such that moving the femoral and tibial components (400, 300) in flexion from about 0° to about 165° causes contact regions between the condyles (402, 401) and bearing surfaces (302, 301) to translate posteriorly at different rates and rotation between the tibial and femoral components (300, 400),
wherein,
the at least one region of uniform radius (α, β, γ) of the medial condyle (402) comprises a first region (α) that engages the medial bearing surface (302) during flexion from about 0° to about 90°,
**characterized in that**
the region of non-uniform radius of the lateral condyle (401) comprises a distal end that engages the lateral bearing surface (301) during flexion from about 0° to about 90°.

2. The prosthesis of claim 1, **characterized in that** the medial and lateral condyles are asymmetrical.

3. The prosthesis of claim 1, **characterized in that** the at least one region of uniform radius (α, β, γ) comprises:
a second region (β) with a second uniform radius where the medial condyle (402) engages the medial bearing surface (302) during flexion from about 90° to about 120°; and
a third region (γ) with a third uniform radius where the medial condyle (402) engages the medial bearing surface (302) during flexion from about 120° to about 165°.

4. The prosthesis of claim 1, **characterized in that** the lateral bearing surface (301) has an anterior projection comprising a lateral anterior lip.

5. The prosthesis of claim 4, **characterized in that** the medial anterior lip (303) has a thickness T_{A} and the lateral anterior lip has a dimension D_{A}.

6. The prosthesis of claim 5, **characterized in that** a proportion of medial anterior lip thickness T_{A} and lateral anterior lip dimension D_{A} is 7 : 4.

7. The prosthesis of any of the preceding claims, **characterized in that** the architecture of the lateral condyle (401) has a changing radius at its distal end.

## Patentansprüche

1. Knieprothese, umfassend eine Femurkomponente (400), welche einen medialen Kondylus (402) und einen lateralen Kondylus (401) mit einer zwischen den zwei Kondylen (402, 401) angeordneten Öffnung (410) aufweist, wobei der mediale Kondylus (402) mindestens einen Bereich von einheitlichem Radius (α, β, γ) aufweist, wo er bei Flexion von ungefähr 0° bis ungefähr 165° an einer medialen Lagerfläche (302) angreift, und der laterale Kondylus (401) einen Bereich von nicht-einheitlichem Radius aufweist, wo er bei Flexion von ungefähr 0° bis ungefähr 165° an einer lateralen Lagerfläche (301) angreift, eine Tibiakomponente (300), welche die mediale Lagerfläche (302) und die laterale Lagerfläche (301) aufweist, um jede der Femurkomponenten-Kondylen (402, 401) zu lagern, wobei die mediale Lagerfläche (302) einen anterioren Vorsprung aufweist, welcher eine medial-anteriore Lippe (303) umfasst, und die Femurkomponente (400) und die Tibiakomponente (300) an Kontaktbereichen zwischen den Femur-Kondylen (402, 401) und den Tibialagerflächen (302, 301) derart in Eingriff bringbar sind, dass bei einer Bewegung der Femur- und Tibiakomponente (400, 300) in Flexion von ungefähr 0° bis ungefähr 90° die medial-anteriore Lippe (303) der medialen Lagerfläche (302) bewirkt, dass der mediale Kondylus (402) an einem konstanten Kontaktpunkt auf der medialen Lagerfläche gehalten wird, und verhindert, dass die Femurkomponente (400) nach vorne rollt, während die laterale Lagerfläche (301) an dem nicht-einheitlichen Radius des lateralen Kondylus (401) angreift, um ein Zurückrollen des lateralen Kondylus an der lateralen Lagerfläche zu ermöglichen, und derart, dass eine Bewegung der Femur- und Tibiakomponente (400, 300) in Flexion von ungefähr 0° bis ungefähr 165° bewirkt, dass Kontaktbereiche zwischen den Kondylen (402, 401) und den Lagerflächen (302, 301) bei zwischen der Tibia- und der Femurkomponente (300, 400) unterschiedlichen Raten und unterschiedlicher Rotation nach posterior translatieren,
wobei
der mindestens eine Bereich von einheitlichem Radius (α, β, γ) des medialen Kondylus (402) einen ersten Bereich (α) umfasst, welcher bei Flexion von ungefähr 0° bis ungefähr 90° an der medialen Lagerfläche (302) angreift,
**dadurch gekennzeichnet, dass**
der Bereich von nicht-einheitlichem Radius des lateralen Kondylus (401) ein distales Ende umfasst, welches bei Flexion von ungefähr 0° bis ungefähr 90° an der lateralen Lagerfläche (301) angreift.

2. Prothese nach Anspruch 1, **dadurch gekennzeichnet, dass** der mediale und der laterale Kondylus asymmetrisch sind.

3. Prothese nach Anspruch 1, **dadurch gekennzeichnet, dass** der mindestens eine Bereich von einheitlichem Radius (α, β, γ) umfasst:
einen zweiten Bereich "(β) mit einem zweiten einheitlichen Radius, wo der mediale Kondylus (402) bei Flexion von ungefähr 90° bis ungefähr 120° an der medialen Lagerfläche (302) angreift; und
einen dritten Bereich (γ) mit einem dritten einheitlichen Radius, wo der mediale Kondylus (402) bei Flexion von ungefähr 120° bis ungefähr 165° an der medialen Lagerfläche (302) angreift.

4. Prothese nach Anspruch 1, **dadurch gekennzeichnet, dass** die laterale Lagerfläche (301) einen anterioren Vorsprung aufweist, welcher eine lateral-anteriore Lippe umfasst.

5. Prothese nach Anspruch 4, **dadurch gekennzeichnet, dass** die medial-anteriore Lippe (303) eine Dicke T_{A} aufweist und die lateral-anteriore Lippe eine Dimension D_{A} aufweist.

6. Prothese nach Anspruch 5, **dadurch gekennzeichnet, dass** ein Verhältnis von Dicke T_{A} der medial-anterioren Lippe und Dimension D_{A} der lateralanterioren Lippe 7:4 beträgt.

7. Prothese nach einem der voranstehenden Ansprüche, **dadurch gekennzeichnet, dass** die Architektur des lateralen Kondylus (401) an dessen distalem Ende einen sich verändernden Radius aufweist.

## Revendications

1. Prothèse de genou comprenant un composant fémoral (400) ayant un condyle médial (402) et un condyle latéral (401) avec une ouverture (410) disposée entre les deux condyles (402, 401), le condyle médial (402) ayant au moins une région de rayon uniforme (α, β, γ) où il engage une surface d'appui médiale (302) durant une flexion d'environ 0° à environ 165°, et le condyle latéral (401) ayant une région de rayon non uniforme où il engage une surface d'appui latérale (301) durant une flexion d'environ 0° à environ 165°, un composant tibial (300) ayant la surface d'appui médiale (302) et la surface d'appui latérale (301) pour supporter chacun des condyles du composant fémoral (402, 401), la surface d'appui médiale (302) ayant une saillie antérieure comprenant une lèvre antérieure médiale (303), et le composant fémoral (400) et le composant tibial (300) pouvant venir en prise au niveau de régions de contact entre les condyles fémoraux (402, 401) et les surfaces d'appui tibiales (302, 301) de façon que, suite au mouvement des composants fémoral et tibial (400, 300) en flexion d'environ 0° à environ 90°, la lèvre antérieure médiale (303) de la surface d'appui médiale (302) provoque le maintien du condyle médial (402) en un point de contact constant sur la surface d'appui médiale et empêche un roulement en direction du composant fémoral (400) tandis que la surface d'appui latérale (301) engage le rayon non uniforme du condyle latéral (401) pour permettre un roulement vers l'arrière du condyle latéral sur la surface d'appui latérale, et de façon que le déplacement des composants fémoral et tibial (400, 300) en flexion d'environ 0° à environ 165° provoque la translation postérieure des régions de contact entre les condyles (402, 401) et des surfaces d'appui (302, 301) à des vitesses et rotations différentes entre les composants tibial et fémoral (300, 400),
dans laquelle l'au moins une région de rayon uniforme (α, β, γ) du condyle médial (402) comprend une première région (α) qui engage la surface d'appui médiale (302) durant une flexion d'environ 0° à environ 90°,
**caractérisée en ce que** la région de rayon non uniforme du condyle latéral (401) comprend une extrémité distale qui engage la surface d'appui latérale (301) durant une flexion d'environ 0° à environ 90°.

2. Prothèse selon la revendication 1, **caractérisée en ce que** les condyles médial et latéral sont asymétriques.

3. Prothèse selon la revendication 1, **caractérisée en ce que** l'au moins une région de rayon non uniforme (α, β, γ) comprend :
une deuxième région (β) avec un deuxième rayon uniforme où le condyle médial (402) engage la surface d'appui médiale (302) durant une flexion d'environ 90° à environ 120° ; et
une troisième région (γ) avec un troisième rayon uniforme où le condyle médial (402) engage la surface d'appui médiale (302) durant une flexion d'environ 120° à environ 165°.

4. Prothèse selon la revendication 1, **caractérisée en ce que** la surface d'appui latérale (301) a une saillie antérieure comprenant une lèvre antérieure latérale.

5. Prothèse selon la revendication 4, **caractérisée en ce que** la lèvre antérieure médiale (303) a une épaisseur T_{A} et la lèvre antérieure latérale a une dimension D_{A}.

6. Prothèse selon la revendication 5, **caractérisée en ce que** le rapport de l'épaisseur de la lèvre antérieure médiale T_{A} à la dimension de la lèvre antérieure latérale D_{A} est de 7/4.

7. Prothèse selon l'une quelconque des revendications précédentes, **caractérisée en ce que** l'architecture du condyle latéral (401) a un rayon changeant à son extrémité distale.
